(19) Europäisches Patentamt
European Patent Office
Office européen des brevets

(11) Publication number: **0 384 285 A2**

(12) **EUROPEAN PATENT APPLICATION**

(21) Application number: **90102889.4**

(22) Date of filing: **14.02.90**

(51) Int. Cl.[5]: **C07D 413/04, C07D 417/04, A61K 31/44**

(30) Priority: **22.02.89 DK 825/89**
**12.05.89 DK 2315/89**

(43) Date of publication of application:
**29.08.90 Bulletin 90/35**

(84) Designated Contracting States:
**AT BE CH DE DK ES FR GB GR IT LI LU NL SE**

(71) Applicant: **A/S FERROSAN**
**Sydmarken 5**
**DK-2860 Soeborg(DK)**

(72) Inventor: **Sauerberg, Per**
**Söndervangsallé 56B**
**DK-2500 valby(DK)**
Inventor: **Olesen, Preben H.**
**Örevadsvej 20**
**DK-2400 København NV(DK)**
Inventor: **Mitch, Charles H.**
**6141 N Sherman Dr.**
**Indianapolis, IN 46220(US)**

(74) Representative: **Patentanwälte Grünecker, Kinkeldey, Stockmair & Partner**
**Maximilianstrasse 58**
**D-8000 München 22(DE)**

(54) **Piperidine compounds and their preparation and use.**

(57) The present invention relates to therapeutically active piperidine compounds, a method of preparing the same and to pharmaceutical compositions comprising the compounds. The novel compounds are useful as stimulants of the cognitive function of the forebrain and hippocampus of mammals and especially in the treatment of Alzheimer's disease.

EP 0 384 285 A2

## Piperidine Compounds and Their Preparation and Use

The present invention relates to therapeutically active piperidine compounds, a method of preparing the same and to pharmaceutical compositions comprising the compounds. The novel compounds are useful as stimulants of the cognitive function of the forebrain and hippocampus of mammals and especially in the treatment of Alzheimer's disease.

Due to the in general improved health situation in the western world, elderly-related diseases are much more common now than in the past and are likely to be even more common in the future.

One of the elderly-related symptoms is a reduction of the cognitive functions. This symptom is especially pronounced in the patophysiological disease known as Alzheimer's disease. This disease is combined with, and also most likely caused by, a up to 90% degeneration of the muscarinic cholinergic neurons in nucleus basalis, which is part of substantia innominata. These neurons project to the prefrontal cortex and hippocampus and have a general stimulatory effect on the cognitive functions of the forebrain as well as of hippocampus, namely learning, association, consolidation, and recognition.

It is a characteristic of Alzheimer's disease that although the cholinergic neurons degenerate, then the postsynaptic muscarinic receptors in the forebrain and hippocampus still exist. Therefore muscarinic cholinergic $M_1$ agonists and $M_2$ antagonists are useful in the treatment of Alzheimer's disease and in improving the cognitive functions of elderly people.

It is well known that arecoline (methyl 1-methyl-1,2,5,6-tetrahydropyridine-3-carboxylate) is such a cholinergic agonist.

Arecoline however has a very short biological half life and a small separation between central and peripheral muscarinic effects. Furthermore arecoline is a rather toxic compound.

U.S. Patent 4,650,805, EP-A-0244018 and DE-A-3703435 disclose certain tetrahydropyridinyl-substituted oxazoles and thiazoles. These compounds are stated to be of use in the treatment of psychosis and disorders of the central nervous system, such as schizophrenia, Parkinson's disease and depression. Said activity does not, however, suggest that any of these compounds could be muscarinic agonists.

In addition EP-A-0261763 and EP-A-0287356 disclose a class of compounds which includes oxazoles and thiazoles having particular exo-azabicyclic substituents. These compounds are stated to be of potential use in the treatment and/or prophylaxis of dementia in mammals.

EP-A-0296721 discloses a class of compounds which includes tetrahydropyridinyl-substituted oxazoles and thiazoles having potent acetylcholine agonist activity, indicating utility in the treatment of diseases caused by reduced function of acetylcholine in the brain.

EP-A-0307141, which was published on March 15, 1989, discloses a class of compounds which includes tetrahydropyridinyl-substituted 1,3-oxazoles and 1,3-thiazoles which stimulate central muscarinic acetylcholine receptors and are therefore useful in the treatment of neurological and mental illnesses, whose clinical manifestations are due to involvement of cholinergic neurones; these compounds are also of benefit in the treatment of severe painful conditions.

It is an object of the invention to provide new muscarinic cholinergic compounds.

The novel compounds of the invention are piperidine compounds having the general formula I

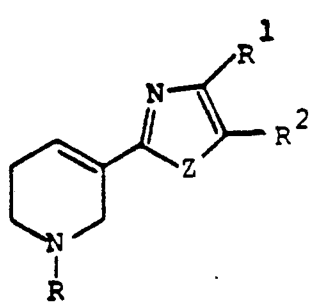

(I)

wherein Z is oxygen or sulphur, R is H, $C_{1-3}$-alkyl, $C_{3-4}$-cycloalkyl, or $C_{2-4}$-alkenyl, or $C_{2-4}$-alkynyl, $R^1$ and $R^2$ independently are $C_{1-10}$-alkyl, $C_{2-10}$-alkenyl, $C_{2-10}$-alkynyl, $C_{1-10}$-alkoxy, $C_{3-10}$-cycloalkyl, $C_{1-10}$-alkylamino, $C_{1-10}$-alkylaminomethyl, $C_{1-10}$-alkoxymethyl, $C_{1-10}$-alkylthio, $C_{1-10}$-alkoxyiminomethyl, halogen or amino, or a salt thereof with a pharmaceutically-acceptable acid.

Examples of such salts include inorganic and organic acid addition salts such as hydrochloride, hydrobromide, sulphate, phosphate, acetate, fumarate, maleate, citrate, lactate, tartrate, oxalate, or similar

pharmaceutically-acceptable inorganic or organic acid addition salt.

The invention also relates to a method of preparing the above mentioned compounds. This method comprises:

a) reacting a compound having the formula II

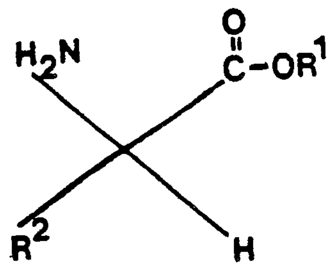

(II)

wherein $R^1$ and $R^2$ have the meanings defined above, with a compound having the formula III

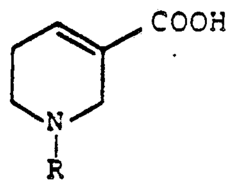

(III)

wherein R has the meaning defined above, to form a compound having the formula IV

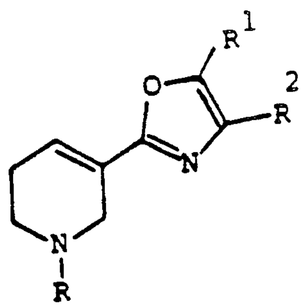

(IV)

wherein R, $R^1$ and $R^2$ have the meanings defined above, or

b) alkylating a compound having the formula V

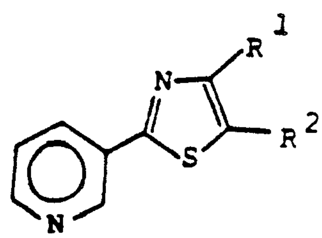

(V)

wherein $R^1$ and $R^2$ have the meanings defined above, with an alkyl halide and reducing the compound thus formed with hydride ions to form a compound having the formula VI

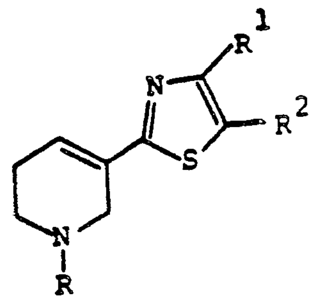

(VI)

wherein R, $R^1$ and $R^2$ have the meanings defined above.

The pharmacological properties of the compounds of the invention can be illustrated by determining their capability to inhibit the specific binding of $^3$H-Oxotremorine-M ($^3$H-Oxo).

$^3$H-Oxo labels muscarinic receptor in the CNS (with a preference for agonist domaines of the receptors). Three different sites are labelled by $^3$H-Oxo. These sites have affinity of 1.8, 20 and 3000 nM, respectively. Using the present experimental conditions only the high and medium affinity sites are determined.

The inhibitory effects of compounds on $^3$H-Oxo binding reflects the affinity for muscarinic acetylcholine receptors.

All preparations are performed at 0-4°C unless otherwise indicated. Fresh cortex (0.1-1 g) from male Wistar rats (150-250 g) is homogenized for 5-10 s in 10 ml 20 mM Hepes pH: 7.4, with an Ultra-Turrax homogenizer. The homogenizer is rinsed with 10 ml of buffer and the combined suspension centrifuged for 15 min at 40,000 $\times$ g. The pellet is washed three times with buffer. In each step the pellet is homogenized as before in $2\times10$ ml of buffer and centrifuged for min at 40,000 $\times$ g.

The final pellet is homogenized in 20 mM Hepes pH: 7.4 (100 ml per g of original tissue) and used for binding assay. Aliquots of 0.5 ml is added 25 µl of test solution and 25 µl of $^3$H-Oxotremorine (1.0 nM, final concentration) mixed and incubated for 30 min at 25°C. Non-specific binding is determined in triplicate using Arecholin (1 µg/ml, final concentration) as the test substance. After incubation samples are added 5 ml of ice-cold buffer and poured directly onto Whatman GF/C glass fiber filters under suction and immediately washed 2 times with 5 ml of ice-cold buffer. The amount of radioactivity on the filters are determined by conventional liquid scintillation counting. Specific binding is total binding minus non specific binding.

Test substances are dissolved in 10 ml water (if necessary heated on a steambath for less than 5 minutes) at a concentration of 2.2 mg/ml. 25-75% inhibition of specific binding must be obtained before calculation of $IC_{50}$.

The test value will be given as $IC_{50}$ (the concentration (ng/ml) of the test substance which inhibits the specific binding of $^3$H-Oxo by 50%).

$$IC_{50} = (\text{applied test susbtance concentration}) \times \frac{1}{\left(\frac{C_o}{C_x} - 1\right)} \text{ ng/ml}$$

where $C_o$ is specific binding in control assays and $C_x$ is the specific binding in the test assay. (The calculations assume normal mass-action kinetics).

Test results obtained by testing some compounds of the present invention will appear from the following table 1.

TABLE 1.

| | Inhibition in vitro OXO BINDING |
|---|---|
| Compound No. | (ng/ml) |
| 1 | 4.5 |
| 2 | 7.9 |
| 3 | 7.9 |
| 4 | 30.2 |
| 5 | 12.0 |
| 6 | 3.7 |
| 7 | 17.0 |
| 8 | 56 |
| 9 | 122 |
| 10 | 54 |
| 11 | 13 |
| 12 | 22 |
| 13 | 67 |
| 14 | 4.5 |
| 15 | 6.9 |
| 16 | 9.2 |
| 17 | 6.9 |
| 18 | 56 |
| 19 | 28 |
| 20 | 46 |
| 21 | 34 |
| 22 | 54 |
| 23 | 28 |

The compounds of the invention, together with a conventional adjuvant, carrier, or diluent, and if desired in the form of a pharmaceutically-acceptable acid addition salt thereof, may be placed into the form of pharmaceutical compositions and unit dosages thereof, and in such form may be employed as solids, such as tablets or filled capsules, or liquids, such as solutions, suspensions, emulsions, elixirs, or capsules filled with the same, all for oral use, in the form of suppositories for rectal administration; or in the form of sterile injectable solutions for parenteral (including subcutaneous) use. Such pharmaceutical compositions and unit dosage forms thereof may comprise conventional ingredients in conventional proportions, with or without additional active compounds or principles, and such unit dosage forms may contain any suitable effective muscarinic cholinergic agonistic amount of the active ingredient commensurate with the intended daily dosage range to be employed. Tablets containing ten (10) milligrams of the active ingredient or, more broadly, one (1) to hundred (100) milligrams, per tablet, are accordingly suitable representative unit dosage forms.

The compounds of this invention can thus be used for the formulation of pharmaceutical preparations, e.g. for oral and parenteral administration to mammals including humans, in accordance with conventional methods of galenic pharmacy.

Conventional excipients are such pharmaceutically acceptable organic or inorganic carrier substances suitable for parenteral or enteral application which do not deleteriously react with the active compounds.

Examples of such carriers are water, salt solutions, alcohols, polyethylene glycols, polyhydroxyethoxylated castor oil, gelatine, lactose, amylose, magnesium stearate, talc, silicic acid, fatty acid monoglycerides and diglycerides, pentaerythritol fatty acid esters, hydroxymethylcellulose and polyvinylpyrrolidone.

The pharmaceutical preparations can be sterilized and mixed, if desired, with auxiliary agents, emulsifiers, salt for influencing osmotic pressure, buffers and/or coloring substances and the like, which do not deleteriously react with the active compounds.

For parenteral application, particularly suitable are injectable solutions or suspensions, preferably aqueous solutions with the active compound dissolved in polyhydroxylated castor oil.

Ampoules are convenient unit dosage forms.

Tablets, dragees, or capsules having talc and/or a carbohydrate carrier or binder or the like, the carrier preferably being lactose and/or corn starch and/or potato starch, are particularly suitable for oral application. A syrup, elixir of the like can be used in cases where a sweetened vehicle can be employed.

Generally, the compounds of this invention are dispensed in unit form comprising 1-100 mg in a pharmaceutically acceptable carrier per unit dosage.

The dosage of the compounds according to this invention is 1-100 mg/day, preferably 10-70 mg/day, when administered to patients, e.g. humans, as a drug.

A typical tablet which may be prepared by conventional tabletting techniques contains:

| | |
|---|---|
| Active compound | 5.0 mg |
| Lactosum | 67.8 mg Ph.Eur. |
| Avicel® | 31.4 mg |
| Amberlite® | 1.0 mg |
| Magnesii stearas | 0.25 mg Ph.Eur. |

Due to the high muscarinic cholinergic receptor agonistic activity, the compounds of the invention are extremely useful in the treatment symptoms related to a reduction of the cognitive functions of the brain of mammals, when administered in an amount effective for stimulating the cognitive functions of the forebrain and hippocampus. The important stimulating activity of the compounds of the invention includes both activity against the patophysiological disease, Alzheimer's disease as well as against normal degeneration of brain function. The compounds of the invention may accordingly be administered to a subject, e.g., a living animal body, including a human, in need of stimulation of the cognitive functions of the forebrain and hippocampus, and if desired in the form of a pharmaceutically-acceptable acid addition salt thereof (such as the hydrobromide, hydrochloride, or sulfate, in any event prepared in the usual or conventional manner, e.g., evaporation to dryness of the free base in solution together with the acid), ordinarily concurrently, simultaneously, or together with a pharmaceutically-acceptable carrier or diluent, especially and preferably in the form of a pharmaceutical composition thereof, whether by oral, rectal, or parenteral (including subcutaneous) route, in an effective forebrain and hippo campus stimulating amount, and in any event an amount which is effective for improving the cognitive function of mammals due to their muscarinic cholinergic receptor agonistic activity. Suitable dosage ranges are 1-100 milligrams daily, 10-100 milligrams daily, and especially 30-70 milligrams daily, depending as usual upon the exact mode of administration, form in which administered, the indication toward which the administration is directed, the subject involved and the body weight of the subject involved, and the preference and experience of the physician or veterinarian in charge.

The invention will now be described in further detail with reference to the following examples:

## EXAMPLE 1

### A. (2S,3S)-2-amino-3-methylpentanoic acid n-propyl ester hydrochloride

Thionyl chloride (27 ml, 360 mmol) was added dropwise to a 0 °C solution of (2S,3S)-2-amino-3-methylpentanoic acid (9.49 g, 72 mmol) in n-propyl alcohol (135 ml, 1800 mmol). The reaction mixture was then heated at 50 °C for 16 h. Upon cooling to room temperature the mixture was evaporated under vacuum. The residue was triturated with cold ethyl ether and filtered. The collected solid was washed thoroughly with cold ethyl ether and dried in a vacuum oven at 40 °C for 16 h.

### B. 1,2,5,6-tetrahydro-3-(5-n-propoxy-4-((S)-1-methylpropyl)-2-oxazolyl)-1-methylpyridine maleate

Oxalyl chloride (7.5 ml, 84.5 mmol) was added dropwise to a 0 °C solution of arecaidine hydrochloride (3 g, 16.9 mmol) in dichloromethane (100 ml). N,N-dimethylformamide (0.1 ml, 1.4 mmol) was then added dropwise to the solution. The reaction mixture was then heated at reflux for 3 h. Upon cooling to room

temperature the solution was evaporated under vacuum. The residue was taken up in dichloromethane (100 ml). To this solution was added (2S,3S)-2-amino-3-methylpentanoic acid n-propyl ester hydrochloride (5 g, 25 mmol) and the resulting mixture was heated at reflux for 48 h. On cooling to room temperature 150 ml saturated sodium bicarbonate was added and the mixture stirred vigorously for 1 h. The phases were separated and the aqueous phase was extracted twice with dichloromethane (150 ml) and phosphoryl chloride (8 ml, 84.5 mmol) added. The reaction mixture was heated at reflux for 24 h. The reaction was then cooled to 0 °C and saturated sodium bicarbonate (150 ml) added and the resulting mixture was stirred vigorously for 1 h. The solution was then adjusted to pH 10 with 50% sodium hydroxide. The phases were separated and the aqueous phase was extracted twice with 150 ml chloroform. The combined organic extracts were dried over sodium sulfate and evaporated under vacuum. The residue was purified by column chromatography ($SiO_2$, eluant chloroform/1% ethanol/0.1% ammonium hydroxide). The title compound was crystallized as the maleate salt from ethyl acetate.
Yield 3.4 g. Mp 115-117 °C. Compound 1.

The following compounds were made in exactly the same manner by reacting arecaidine hydrochloride with amino esters prepared from the corresponding alcohols and amino acids.

1,2,5,6-tetrahydro-3-(5-ethoxy-4-methyl-2-oxazolyl)-1-methylpyridine maleate. Mp 130-132 °C. Compound 2.

1,2,5,6-tetrahydro-3-(5-methoxy-4-(2-methylpropyl)-2-oxazolyl)-1-methylpyridine maleate. Mp 118-120 °C. Compound 3.

1,2,5,6-tetrahydro-3-(5-methoxy-4-ethyl-2-oxazolyl)-1-methylpyridine maleate. Mp 95-97 °C. Compound 4.

1,2,5,6-tetrahydro-3-(5-methoxy-4-n-butyl-2-oxazolyl)-1methylpyridine maleate. Mp 96-98 °C. Compound 5.

1,2,5,6-tetrahydro-3-(5-methoxy-4-((S)-1-methylpropyl)-2-oxazolyl)-1-methylpyridine maleate. Mp 93-95 °C. Compound 6.

1,2,5,6-tetrahydro-3-(5-methoxy-4-i-propyl-2-oxazolyl)-1-methylpyridine maleate. Mp 117-119 °C. Compound 7.

1,2,5,6-tetrahydro-3-(5-methoxy-4-benzyl-2-oxazolyl)-1-methylpyridine maleate. Mp 210-212 °C. Compound 8.

1,2,5,6-tetrahydro-3-(5-methoxy-4-(2-methylthioethyl)-2-oxazolyl)-1-methylpyridine maleate. Mp 73-75 °C. Compound 9.

1,2,5,6-tetrahydro-3-(5-methoxy-4-methyl-2-oxazolyl)-1-methylpyridine maleate. Mp 103-105 °C. Compound 10.

1,2,5,6-tetrahydro-3-(5-propoxy-4-methyl-2-oxazolyl)-1-methylpyridine maleate. Mp 93-95 °C. Compound 11.

1,2,5,6-tetrahydro-3-(5-ethoxy-4-(2-methylpropyl)-2-oxazolyl)-1-methylpyridine maleate. Mp 107-109 °C. Compound 12.

1,2,5,6-tetrahydro-3-(5-propoxy-4-(2-methylpropyl)-2-oxazolyl)-1-methylpyridine maleate. Mp 98-100 °C. Compound 13.

1,2,5,6-tetrahydro-3-(5-ethoxy-4-((S)-1-methylpropyl)-2-oxazolyl)-1-methylpyridine maleate. Mp 92-94 °C. Compound 14.

1,2,5,6-tetrahydro-3-(5-butoxy-4-((S)-1-methylpropyl)-2-oxazolyl)-1-methylpyridine maleate. Mp 117-119 °C. Compound 15.

1,2,5,6-tetrahydro-3-(5-methoxy-4-((R)-1-methylpropyl)-2-oxazolyl)-1-methylpyridine maleate. Mp 89-91 °C. Compound 16.

1,2,5,6-tetrahydro-3-(5-isopropoxy-4-(2-methylpropyl)-2-oxazolyl)-1-methylpyridine maleate. Mp 89-91 °C. Compound 17.

## EXAMPLE 2

### A. 3-(4-chloro-5-formyl-2-thiazolyl)pyridine

Dry dimethylformamide (40 ml, 0.51 mol) was cooled to 0-5 °C with an ice/water bath. Phosphorus oxychloride (40 ml, 0.43 mol) was added dropwise over 1 h. 3-(4-hydroxy-2-thiazolyl)pyridine hydrobromide (26 g, 0.105 mol) was added in one portion and the reaction mixture was heated to 110 °C for 15 min. After cooling the reaction mixture was poured on ice water (1000 ml) and neutralized with solid potassium carbonate. The precipitate was filtered, washed with water (3x50 ml), once with ethanol (20 ml), and dried. Yield 17.2 g, 73%. Mp 160-61 °C.

B. 3-(5-formyl-4-methoxy-2-thiazolyl)pyridine

3-(4-chloro-5-formyl-2-thiazolyl)pyridine (11.2 g, 0.05 mol) was added to a solution of sodium (2.2 g, 0.10 mol) in methanol (75 ml). The reaction mixture was heated at refluxing temperature for 2 h. After cooling the reaction mixture was poured on ice water (100 ml). The precipitate was filtered, washed with water and dried. Yield 8.9 g, 81%. Mp 166-67 °C.

C. 3-(4-methoxy-5-cyclopropylmethoxyiminomethyl-2-thiazolyl)-1,2,5,6-tetrahydro-1-methylpyridine oxalate

To a suspension of 3-(4-methoxy-5-formyl-2-thiazolyl)pyridine (1.1 g, 5 mmol) in methanol (20 ml) was added O-cyclopropylmethylhydroxylamine hydrochloride (0.75 g, 6 mmol) and triethylamine (1 ml). The reaction mixture was stirred at room temperature for 3 days. After concentration in vacuo water (50 ml) was added and the water phase was extracted with ether (3 x 30 ml). The combined ether extracts were dried over magnesium sulphate and concentrated in vacuo. The residue was dissolved in acetone (30 ml) and methyl iodide (4.5 g, 30 mmol) was added. The reaction mixture was stirred at room temperature for 2 days, then concentrated in vacuo and redissolved in methanol (40 ml). Sodium borohydride (0.76 g, 20 mmol) was added to the reaction mixture over 0.5 h. After concentration in vacuo water (50 ml) was added and the water phase extracted with ether (3 x 40 ml). The combined ether extracts were dried over magnesium sulphate and evaporated in vacuo. The title compound was finally purified by column chromatography with acetone as eluent. Crystallization of the residue with oxalic acid from acetone/ether gave the title compound in 32% (700 mg) yield. Mp 184-185 °C. Compound 18.

In exactly the same manner the following compounds were prepared, starting from 3-(4-methoxy-5-formyl-2-thiazolyl)pyridine and the appropriate O-alkylhydroxylamine hydrochloride.

3-(4-methoxy-5-methoxyiminomethyl-2-thiazolyl)-1,2,5,6-tetrahydro-1-methylpyridine oxalate. Mp 178-180 °C. Compound 19.

3-(4-methoxy-5-butoxyiminomethyl-2-thiazolyl)-1,2,5,6-tetrahydro-1-methylpyridine oxalate. Mp 131-133 °C. Compound 20.

3-(4-methoxy-5-isopropoxyiminomethyl-2-thiazolyl)-1,2,5,6-tetrahydro-1-methylpyridine oxalate. Mp 145-147 °C. Compound 21.

## EXAMPLE 3

3-(5-hydroxymethyl-4-methoxy-2-thiazolyl)-1,2,5,6-tetrahydro-1-methylpyridine oxalate

To a suspension of 3-(5-formyl-4-methoxy-2-thiazolyl)pyridine (1.1 g, 5 mmol) in acetone (40 ml) was added methyl iodide (4.5 g, 30 mmol). The reaction mixture was stirred at room temperature for 2 days, then concentrated in vacuo and redissolved in methanol (30 ml). Sodium borohydride (0.76 g, 20 mmol) was slowly added to the reaction mixture. After concentration in vacuo water (50 ml) was added and the water phase extracted with ether (3 x 40 ml). The combined ether extracts were dried over magnesium sulphate and evaporated in vacuo. The title compound was finally purified by column chromatography with acetone as eluent. Crystallization of the residue with oxalic acid from acetone gave the title compound in 34% (560 mg) yield. Mp 127-128 °C. Compound 22.

## EXAMPLE 4

A. 3-(5-hydroxymethyl-4-methoxy-2-thiazolyl)pyridine

To a suspension of 3-(5-formyl-4-methoxy-2-thiazolyl)pyridine (2.2 g, 10 mmol) in methanol (30 ml) was added sodium borohydride (0.38 g, 10 mmol) in 0.5 h. Water (50 ml) was added and the precipitated crystals were filtered, washed with water and dried. Yield 100% (2.2 g). Mp 108-110 °C.

B. 3-(4-methoxy-5-methoxymethyl-2-thiazolyl)-1,2,5,6-tetrahydro-1-methylpyridine oxalate

To a suspension of sodium hydride (0.24 g, 55% in oil, 5.5 mmol) in tetrahydrofuran (20 ml) was added 3-(5-hydroxymethyl-4-methoxy-2-thiazolyl)pyridine (1.1 g, 5 mmol). The reaction mixture was stirred at room temperature for 0.5 h. Methyl iodide (0.71 g, 5 mmol) was added and the reaction mixture stirred at room temperature for 4 h. Methyl iodide (4.5 g, 30 mmol) was added and the mixture was stirred at room temperature for another 2 days, then concentrated in vacuo and redissolved in methanol (30 ml). Sodium borohydride (0.76 g, 20 mmol) was slowly added to the reaction mixture. After concentration in vacuo, water (50 ml) was added and the water phase extracted with ether (3 x 40 ml). The combined ether extracts were dried over magnesium sulphate and evaporated in vacuo. The title compound was finally purified by column chromatography with acetone as eluent. Crystallization of the residue with oxalic acid from ether/ethanol gave the title compound in 25% (400 mg) yield. Mp 125-127° C. Compound 23.

## Claims

1. A compound of formula I

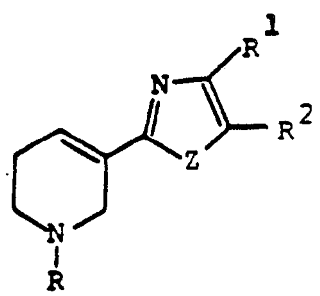

(I)

wherein Z is oxygen or sulphur, R is H, $C_{1-3}$-alkyl, $C_{3-4}$-cycloalkyl, $C_{2-4}$-alkenyl or $C_{2-4}$-alkynyl, $R^1$ and $R^2$ independently are $C_{1-10}$-alkyl, $C_{2-10}$-alkenyl, $C_{2-10}$-alkynyl, $C_{1-10}$-alkoxy, $C_{3-10}$-cycloalkyl, $C_{1-10}$-alkylamino, $C_{1-10}$-alkykaminomethyl, $C_{1-10}$-alkylmethyl, $C_{1-10}$-alkylthio, $C_{1-10}$-alkoxyiminomethyl, halogen or amino, or a salt thereof with a pharmaceutically-acceptable acid.

2. A compound selected from the following:
1,2,5,6-tetrahydro-3-(5-n-propoxy-4-((S)-1-methylpropyl)-2-oxazolyl)-1-methylpyridine:
1,2,5,6-tetrahydro-3-(5-ethoxy-4-methyl-2-oxazolyl)-1-methylpyridine;
1,2,5,6-tetrahydro-3-(5-methoxy-4-(2-methylpropyl)-2-oxazolyl)-1-methylpyridine;
1,2,5,6-tetrahydro-3-(5-methoxy-4-ethyl-2-oxazolyl)-1-methylpyridine;
1,2,5,6-tetrahydro-3-(5-methoxy-4-n-butyl-2-oxazolyl)-1-methylpyridine;
1,2,5,6-tetrahydro-3-(5-methoxy-4-((S)-1-methylpropyl)-2-oxazolyl)-1-methylpyridine;
1,2,5,6-tetrahydro-3-(5-methoxy-4-i-propyl-2-oxazolyl)-1-methylpyridine;
1,2,5,6-tetrahydro-3-(5-methoxy-4-benzyl-2-oxazolyl)-1-methylpyridine;
1,2,5,6-tetrahydro-3-(5-methoxy-4-(2-methylthioethyl)-2-oxazolyl)-1-methylpyridine;
1,2,5,6-tetrahydro-3-(5-methoxy-4-methyl-2-oxazolyl)-1-methylpyridine;
1,2,5,6-tetrahydro-3-(5-propoxy-4-methyl-2-oxazolyl)-1-methylpyridine;
1,2,5,6-tetrahydro-3-(5-ethoxy-4-(2-methylpropyl)-2-oxazolyl)-1-methylpyridine;
1,2,5,6-tetrahydro-3-(5-propoxy-4-(2-methylpropyl)-2-oxazolyl)-1-methylpyridine;
1,2,5,6-tetrahydro-3-(5-ethoxy-4-((S)-1-methylpropyl)-2-oxazolyl)-1-methylpyridine;
1,2,5,6-tetrahydro-3-(5-butoxy-4-((S)-1-methylpropyl)-2-oxazolyl)-1-methylpyridine;
1,2,5,6-tetrahydro-3-(5-methoxy-4-((R)-1-methylpropyl)-2-oxazolyl)-1-methylpyridine;
1,2,5,6-tetrahydro-3-(5-isopropoxy-4-(2-methylpropyl)-2-oxazolyl)-1-methylpyridine;
3-(4-methoxy-5-cyclopropylmethoxyiminomethyl-2-thiazolyl)-1,2,5,6-tetrahydro-1-methylpyridine;
3-(4-methoxy-5-methoxyiminomethyl-2-thiazolyl)-1,2,5,6-tetrahydro-1-methylpyridine;
3-(4-methoxy-5-butoxyiminomethyl-2-thiazolyl)-1,2,5,6-tetrahydro-1-methylpyridine;
3-(4-methoxy-5-isopropoxyiminomethyl-2-thiazolyl)-1,2,5,6-tetrahydro-1-methylpyridine;
3-(5-hydroxymethyl-4-methoxy-2-thiazolyl)-1,2,5,6-tetrahydro-1-methylpyridine;
3-(4-methoxy-5-methoxymethyl-2-thiazolyl)-1,2,5,6-tetrahydro-1-methylpyridine;
and salts thereof with pharmaceutically-acceptable acids.

3. A method of preparing a compound according to claim 1, CHARACTERIZED IN:

a) reacting a compound having the formula II

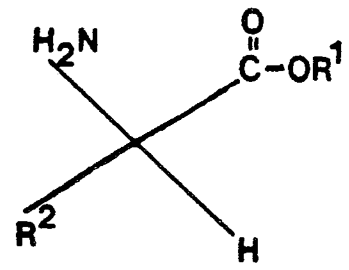

(II)

wherein $R^1$ and $R^2$ have the meanings defined above, with a compound having the formula III

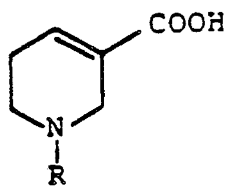

(III)

wherein R has the meaning defined above, to form a compound having the formula IV

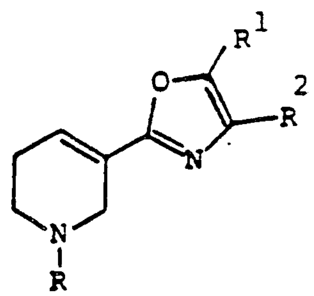

(IV)

wherein R, $R^1$ and $R^2$ have the meanings defined above, or

b) alkylating a compound having the formula V

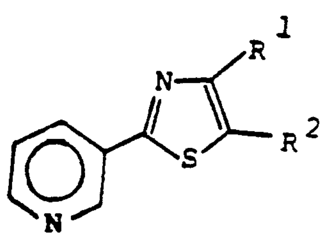

(V)

wherein $R^1$ and $R^2$ have the meanings defined above, with an alkyl halide and reducing the compound thus formed with hydride ions to form a compound having the formula VI

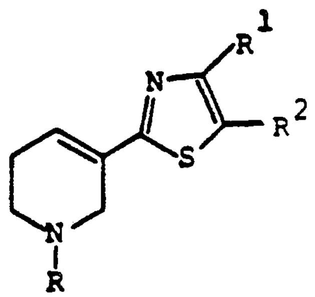

(VI)

wherein R, $R^1$ and $R^2$ have the meanings defined above.

4. A pharmaceutical composition suitable for use in stimulating the cognitive functions of the forebrain and hippocampus of mammals, including humans, and in treating Alzheimer's disease, comprising an amount of a compound of formula I wherein Z is oxygen or sulphur, R is H, $C_{1-3}$-alkyl, $C_{3-4}$-cycloalkyl, $C_{2-4}$-alkenyl or $C_{2-4}$-alkynyl, $R^1$ and $R^2$ independently are $C_{1-10}$-alkyl, $C_{2-10}$-alkenyl, $C_{2-10}$-alkynyl, $C_{1-10}$-alkoxy, $C_{3-10}$-cycloalkyl, $C_{1-10}$-alkylamino, $C_{1-10}$-alkylaminomethyl, $C_{1-10}$-alkoxymethyl, $C_{1-10}$-alkylthio, $C_{1-10}$-alkoxyiminomethyl, halogen or amino, or a salt thereof with a pharmaceutically-acceptable acid which compound is effective for the stimulation of the forebrain and hippocampus of mammals or treating Alzheimer's disease together with a pharmaceutically-acceptable carrier or diluent.

5. The pharmaceutical composition according to claim 4 in the form of an oral dosage unit containing 1-100 mg of the compound of formula I or a salt thereof with a pharmaceutically-acceptable acid.

6. A method of stimulating the cognitive functions of the forebrain and hippocampus and therefore of treating Alzheimer's disease in a subject in need of such stimulation and/or treatment, comprising the step of administering to said subject an amount of a compound of formula I

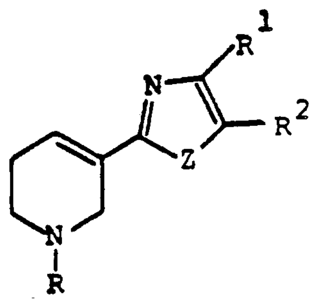

(I)

wherein Z is oxygen or sulphur, R is H, $C_{1-3}$-alkyl, $C_{3-4}$-cycloalkyl, $C_{2-4}$-alkenyl or $C_{2-4}$-alkynyl, $R^1$ and $R^2$ independently are $C_{1-10}$-alkyl, $C_{2-10}$-alkenyl, $C_{2-10}$-alkynyl, $C_{1-10}$-alkoxy, $C_{3-10}$-cycloalkyl, $C_{1-10}$-alkylamino, $C_{1-10}$-alkylaminomethyl, $C_{1-10}$-alkoxymethyl, $C_{1-10}$-alkylthio, $C_{1-10}$-alkoxyiminomethyl, halogen or amino, or a salt thereof with a pharmaceutically-acceptable acid which compound is effective for such purpose.

7. A method of claim 6 wherein said compound is administered in the form of a pharmaceutical composition together with a pharmaceutically-acceptable carrier or diluent.